# EUROPEAN PATENT APPLICATION

(11) **EP 4 342 383 A1**
(43) Date of publication of application: **27.03.2024**
(21) Application number: 22946046.4
(22) Date of filing: 06.07.2022
(51) Int. Cl.: A61B 6/00, A61B 5/055, H01R 39/08

(54) **SLIDABLE RING ASSEMBLY AND MEDICAL DEVICE WITH SAME**

(71) Applicant: Shanghai United Imaging Healthcare Co., Ltd., Shanghai 201807 (CN)
(72) Inventor: WANG, Peng, Shanghai 201807 (CN); ZOU, Jianxiong, Shanghai 201807 (CN); FU, Feichao, Shanghai 201807 (CN); NI, Cheng, Shanghai 201807 (CN)
(74) Representative: Murgitroyd & Company
(86) International application number: PCT/CN2022/104244
(87) International publication number: WO 2024/007229

(57) **Abstract**

A slip ring assembly (300) includes: at least one first conductor group, and at least one second conductor group. Each first conductor group includes a first conductor (310) and a second conductor (320), and the first conductor (310) and the second conductor (320) are configured as annular structures, respectively. Each second conductor group includes a third conductor (330) and a fourth conductor (340), the third conductor (330) and the fourth conductor (340) are configured as annular structures, respectively. The first conductor (310), the second conductor (320), the third conductor (330) and the fourth conductor (340) are arranged to be apart from each other. A first current having a first phase and a first amplitude is configured to flow through the first conductor (310), a second current having a second phase and a second amplitude is configured to flow through the second conductor (320), a third current having a third phase and a third amplitude is configured to flow through the third conductor (330), and a fourth current having a fourth phase and a fourth amplitude is configured to flow through the fourth conductor (320).

## Description

### TECHNICAL FIELD

The present application relates to the field of magnetic resonance medical technology, and in particular to a slip ring assembly, and a medical device having the slip ring assembly.

### BACKGROUND

In modem medicine, imaging equipment is usually used to obtain images of patients to make it easy for a doctor to diagnose or to treat patients by using treatment equipment. For example, magnetic resonance imaging devices are used to obtain magnetic resonance images of tissues of patients (such as tumor patients) to clearly display the conditions of the diseased site and surrounding tissues, so that the diseased tissue can be accurately located. Further, a precise radiation therapy is performed by using the treatment equipment (such as an electronic linear accelerator) based on magnetic resonance images. Magnetic resonance and the treating device may be used in combination, and the slip ring assembly may be used as a power supply medium and a communication medium of the treating device. When the slip ring assembly is powered on, the magnetic field generated by the current flowing through the slip ring assembly will interfere with a central magnetic field of a magnetic resonance imaging (MRI) device, thus resulting in a lower accuracy of the MRI.

### SUMMARY

Based on this, in order to address the above technical problems, it is necessary to provide a slip ring assembly and a medical device having the slip ring assembly, which can effectively reduce an interference of a magnetic field generated by a current flowing through the slip ring assembly in the central magnetic field of the (MRI) device.

According to one aspect of the present application, a slip ring assembly is provided and includes: at least one first conductor group and at least one second conductor group. Each first conductor group includes a first conductor and a second conductor, and the first conductor and the second conductor are configured as annular structures, respectively. Each second conductor group includes a third conductor and a fourth conductor, the third conductor and the fourth conductor are configured as annular structures, respectively. The first conductor, the second conductor, the third conductor and the fourth conductor are arranged to be apart from each other. A first current having a first phase and a first amplitude is configured to flow through the first conductor, a second current having a second phase and a second amplitude is configured to flow through the second conductor, a third current having a third phase and a third amplitude is configured to flow through the third conductor, and a fourth current having a fourth phase and a fourth amplitude is configured to flow through the fourth conductor. A difference between the first phase and the third phase is less than or equal to a first threshold, and/or a difference between the first amplitude and the third amplitude is less than or equal to a second threshold. A difference between the second phase and the fourth phase is less than or equal to a third threshold, and/or a difference between the second amplitude and the fourth amplitude is less than or equal to a fourth threshold. A first magnetic field generated by the first conductor group and a second magnetic field generated by the second conductor group at least partially offset each other.

In a slip ring assembly provided in the present application, the first magnetic field generated by the first conductor group and the second magnetic field generated by the second conductor group at least partially offset each other after superposed in space, and therefore the overall intensity of the magnetic field (an interference magnetic field) generated in the slip ring assembly is reduced, thereby reducing the interference in the central magnetic field of the MRI device, and ensuring the accuracy of the MRI.

In an embodiment, the first threshold and/or the third threshold are 60°.

In an embodiment, the second threshold is twice a smaller one of the first amplitude and the third amplitude, and/or the fourth threshold is twice a smaller one of the second amplitude and the fourth amplitude.

In an embodiment, the first conductor group and the second conductor group are coaxially arranged.

In an embodiment, the first conductor and the second conductor are coaxially arranged.

In an embodiment, the third conductor and the fourth conductor are coaxially arranged.

In an embodiment, the first conductor group and the second conductor group are arranged at different positions along an axial direction (X).

In an embodiment, the first conductor and the second conductor are arranged at the same position along the axial direction (X), and the third conductor and the fourth conductor are arranged at the same position along the axial direction (X).

In an embodiment, a radial dimension of the first conductor is greater than a radial dimension of the second conductor, and a radial dimension of the fourth conductor is greater than a radial dimension of the third conductor.

In an embodiment, the radial dimension of the first conductor is equal to the radial dimension of the fourth conductor, and the radial dimension of the second conductor is equal to the radial dimension of the third conductor.

In an embodiment, the first conductor and the second conductor are arranged at different positions along the axial direction (X), and the third conductor and the fourth conductor are arranged at different positions along the axial direction (X).

In an embodiment, the first conductor, the second conductor, the fourth conductor, and the third conductor are arranged in sequence along an axial direction (X).

In an embodiment, a radial dimension of the first conductor, a radial dimension of the second conductor, a radial dimension of the fourth conductor and a radial dimension of the third conductor are the same.

In an embodiment, the first conductor group and the second conductor group are arranged at the same position along an axial direction (X).

In an embodiment, the first conductor and the second conductor are arranged at the same position along the axial direction (X), and the third conductor and the fourth conductor are arranged at the same position along the axial direction (X).

In an embodiment, a radial dimension of the first conductor, a radial dimension of the second conductor, a radial dimension of the fourth conductor, and a radial dimension of the third conductor increase in sequence.

In an embodiment, the first conductor and the second conductor are arranged at different positions in a first direction along an axial direction (X), and the fourth conductor and the third conductor are arranged at different positions in the first direction along the axial direction (X).

In an embodiment, a smaller one of a radial dimension of the first conductor and a radial dimension of the second conductor is greater than a larger one of a radial dimension of the third conductor and a radial dimension of the fourth conductor.

In an embodiment, the radial dimension of the first conductor is equal to the radial dimension of the second conductor, and the radial dimension of the third conductor is equal to the radial dimension of the fourth conductor.

In an embodiment, the first conductor group further includes a fifth conductor, and the fifth conductor is configured to be in an annular shape; the second conductor group further includes a sixth conductor, and the sixth conductor is configured to be in an annular shape. The first conductor, the second conductor, the third conductor, the fourth conductor, the fifth conductor, and the sixth conductor are configured to be apart from each other. A fifth current having a fifth phase and a fifth amplitude is configured to pass through the fifth conductor, and a sixth current having a sixth phase and a sixth amplitude is configured to pass through the sixth conductor. A difference between the fifth phase and the sixth phase is less than or equal to a fifth threshold, or a difference between the fifth amplitude and the sixth amplitude is less than or equal to a sixth threshold.

In an embodiment, the fifth threshold is 60°.

In an embodiment, the sixth threshold is twice a smaller one of the fifth amplitude and the sixth amplitude.

In an embodiment, the first conductor, the second conductor and the fifth conductor are coaxially arranged.

In an embodiment, the third conductor, the fourth conductor, and the sixth conductor are coaxially arranged.

In an embodiment, at least two of the first conductor, the second conductor and the fifth conductor are arranged at the same position along an axial direction (X), and at least two of the third conductor, the fourth conductor and the sixth conductor are arranged at the same position along the axial direction (X).

In an embodiment, the first conductor, the second conductor and the fifth conductor are arranged at the same position along the axial direction (X), and the third conductor, the fourth conductor and the sixth conductor are arranged at the same position along the axial direction (X).

In an embodiment, a radial dimension of the first conductor, a radial dimension of the second conductor and a radial dimension of the fifth conductor decrease in sequence, and a radial dimension of the sixth conductor, a radial dimension of the fourth conductor, and a radial dimension of the third conductor decrease in sequence.

In an embodiment, the radial dimension of the first conductor is equal to the radial dimension of the sixth conductor, the radial dimension of the second conductor is equal to the radial dimension of the fourth conductor, and the radial dimension of the fifth conductor is equal to the radial dimension of the third conductor.

In an embodiment, at least two of the first conductor, the second conductor and the fifth conductor are arranged at different positions along an axial direction (X), and at least two of the third conductor, the fourth conductor and the sixth conductor are arranged different positions along the axial direction (X).

In an embodiment, the first conductor, the second conductor and the fifth conductor are arranged at different positions along the axial direction (X), and the third conductor, the fourth conductor and the sixth conductor are arranged at different positions along the axial direction (X).

In an embodiment, the fifth conductor, the second conductor, the first conductor, the third conductor, the fourth conductor and the sixth conductor are arranged in sequence along an axial direction (X).

In an embodiment, at least two of the first conductor, the second conductor and the fifth conductor are arranged at the same position along an axial direction (X), and at least two of the third conductor, the fourth conductor and the sixth conductor are at the same position along the axial direction (X).

In an embodiment, the first conductor, the second conductor and the fifth conductor are arranged at the same position along the axial direction (X), the third conductor, the fourth conductor and the sixth conductor are arranged at the same position along the axial direction (X).

In an embodiment, a radial dimension of the fifth conductor, a radial dimension of the second conductor, a radial dimension of the first conductor, a radial dimension of the third conductor, a radial dimension of the fourth conductor, and a radial dimension of the sixth conductor increase in sequence.

In an embodiment, at least two of the first conductor, the second conductor and the fifth conductor are arranged at different positions along an axial direction (X), and at least two of the third conductor, the fourth conductor and the sixth conductor are arranged at different positions along the axial direction (X).

In an embodiment, the first conductor, the second conductor and the fifth conductor are arranged at different positions in a second direction along an axial direction (X); and the third conductor, the fourth conductor and the sixth conductor are arranged at different positions in the second direction along the axial direction (X).

In an embodiment, the smallest one of a radial dimension of the first conductor, a radial dimension of the second conductor and a radial dimension of the fifth conductor is greater than the largest one of a radial dimension of the third conductor, a radial dimension of the fourth conductor, and a radial dimension of the sixth conductor.

In an embodiment, the slip ring assembly further includes a first brush, a second brush, a third brush and a fourth brush, the first brush is adapted to form an electrical contact with the first conductor, the second brush is adapted to form an electrical contact with the second conductor, the third brush is adapted to form an electrical contact with the third conductor, and the fourth brush is adapted to form an electrical contact with the fourth conductor.

In an embodiment, the slip ring assembly further includes a fifth brush and a sixth brush, wherein the fifth brush is adapted to form an electrical contact with the fifth conductor, and the sixth brush is adapted to form an electrical contact with the sixth conductor.

According to another aspect of the invention, a medical device is provided and includes a treating device. The treating device includes the slip ring assembly of any embodiment above, and the slip ring assembly is adapted to act as a power supply medium or a communication medium of the treating device.

In an embodiment, the medical device further includes a magnetic resonance imaging device.

### BRIEF DESCRIPTION OF THE DRAWINGS

The above and other objectives, features and advantages of the present invention will become more apparent through a more particular description of the preferred embodiments of the present invention as shown in the accompanying drawings. The same reference numerals refer to the same parts throughout the drawings. The drawings are not intentionally drawn to scale to actual size, but intended to emphasize and illustrate the subject of the present invention.

Other features, objectives and advantages of the present invention will become more apparent upon reading the detailed description of the non-limiting embodiments with reference to the following drawings:
FIG. 1 is a perspective diagram of a medical device according to an embodiment of the present application;
FIG. 2 is a perspective diagram of a slip ring assembly according to an embodiment of the present application;
FIG. 3 is a schematic sectional view of the slip ring assembly shown in FIG. 2;
FIG. 4 is a perspective diagram of the slip ring assembly according to an embodiment of the present application;
FIG. 5a is a perspective diagram of the slip ring assembly according to an embodiment of the present application;
FIG. 5b is a schematic sectional view of the slip ring assembly shown in FIG. 5a;
FIG. 6a is a perspective diagram of the slip ring assembly according to an embodiment of the present application;
FIG. 6b is a schematic sectional view of the slip ring assembly shown in FIG. 6a;
FIG. 7 is a perspective diagram of a slip ring assembly according to an embodiment of the present application;
FIG. 8 is a schematic sectional view of the slip ring assembly shown in FIG. 7;
FIG. 9 is a perspective diagram of the slip ring assembly according to an embodiment of the present application;
FIG. 10 is a perspective diagram of the slip ring assembly according to an embodiment of the present application; and
FIG. 11 is a schematic sectional view of the slip ring assembly shown in FIG. 10.

### DETAILED DESCRIPTION OF THE EMBODIMENTS

In order to facilitate understanding of the present application, the present application will be described more completely hereinafter with reference to the relevant drawings. Preferred embodiments of the present application are shown in the accompanying drawings, however, the present application may be implemented in many different forms and is not limited to the embodiments described herein. Rather, these embodiments are provided so as to make the disclosure of the present application more thorough and complete.

It should be noted that, when an element is referred to as "connected" to another element, it may be directly connected to and integral with the other element, or there may be intervening elements therebetween. The terms "installation", "one end", "another end", and similar expressions used herein are for illustrative purposes only.

Unless otherwise defined, all technical and scientific terms used herein have the same meaning as commonly understood by one of the ordinary skill in the technical field of this application. The terminology used herein in the description of the application is for the purpose of describing specific embodiments only and is not intended to limit the application. The term "and/or" herein includes any and all combinations of one or more of the associated listed items.

In order to make the purpose, technical solutions and advantages of the present application clearer, the present application will be further described in detail hereinafter with reference to the drawings and embodiments. It should be understood that the specific embodiments described herein are only used to explain the present application, but not intended to limit the present application.

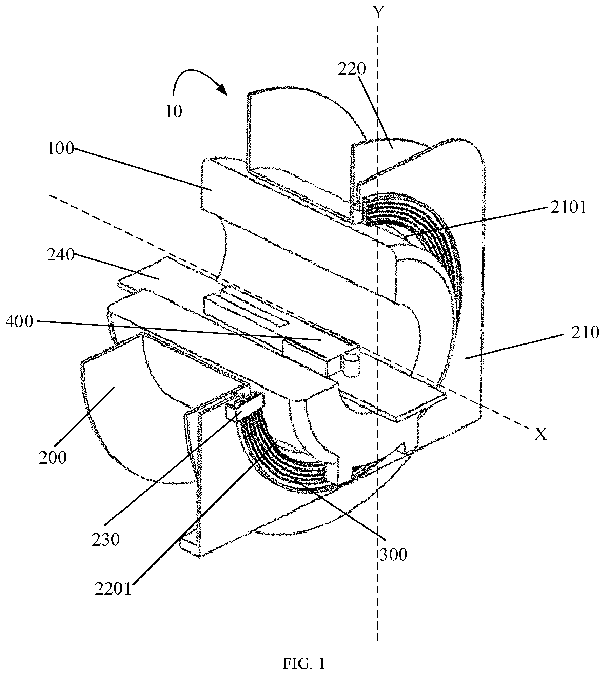

Referring to FIG. 1, FIG. 1 is a perspective diagram of a medical device 10 according to an embodiment of the present application. In this embodiment, the medical device 10 includes: a magnetic resonance imaging (MRI) device 100, a treating device 200, and a bed assembly 240. The MRI device 100 is configured to be in a cylindrical shape and have a hollow portion. The treating device 200 is, for example, a linear accelerator 200. Optionally, the treating device 200 may also be any other suitable treating device, which is not limited in this application. The linear accelerator 200 includes: an accelerator stator 210, an accelerator roller 220, and an electrically connecting terminal 230. The accelerator stator 210 is fixedly arranged, and the accelerator stator 210 has a circular first opening 2101. The accelerator roller 220 has a circular second opening 2201, the second opening 2201 and the first opening 2101 are arranged coaxially, and the accelerator roller 220 is configured to be pivotable around the common axis of thereof relative to the accelerator stator 210. The slip ring assembly 300 is provided on one side of the accelerator roller 210, and the slip ring assembly 300 is arranged coaxially with the first opening 2101 and the second opening 2201. In FIG. 1, an X-axis and a Y-axis illustratively show an axial direction and a radial direction of the slip ring assembly 300 respectively. The electrically connecting terminal 230 is arranged on the accelerator stator 210 and may be electrically connected to the slip ring assembly 300. In this embodiment, the electrically connecting terminal 230 is, for example, a carbon brush 230. Optionally, the electrically connecting terminal 230 may also be any other suitable electrical connection device, such as a metal brush, etc., which is not limited in the application, as long as it may be ensured that the slip ring assembly 300 is electrically connected to the linear accelerator 200. In this embodiment, the slip ring assembly 300 is arranged on the accelerator roller 220, and the electrically connecting terminal 230 is arranged on the accelerator stator 210. When the linear accelerator 200 operates, the slip ring assembly 300 pivots together with the accelerator roller 210, and the slip ring assembly 300 is electrically connected to the linear accelerator 200 through the carbon brush 230. Those skilled in the art may understand that the slip ring assembly 300 and the electrically connecting terminal 230 may also be arranged at other suitable positions, as long as it may be achieved that the slip ring assembly 300 and the electrically connecting terminal 230 may slide relative to each other, and that the slip ring assembly 300 may be electrically connected to the linear accelerator 200 through the carbon brush 230. The installation position of the slip ring assembly 300 and the installation position of the electrically connecting terminal 230 are not limited in the present application. For example, optionally, the slip ring assembly 300 may also be arranged on the accelerator stator 210, and the electrically connecting terminal 230 is arranged on the accelerator roller 220. When the linear accelerator 200 works, the carbon brush 230 pivots together with the accelerator roller 210, and the slip ring assembly 300 is electrically connected to the linear accelerator 200 through the carbon brush 230. The bed assembly 240 is configured to be at least partially moved into the hollow portion of the MRI device 100, and includes a bed board adapted to carry a patient 400 to be examined or to be treated. When the medical device 10 is working, the patient 400 is positioned on the bed board of the bed assembly 240, and the MRI device 100 is adapted to perform MRI on the region of interest (ROI) of the patient 400, to obtain magnetic resonance images. The linear accelerator 200 is adapted to perform a radiotherapy on the lesion tissue within the ROI based on the magnetic resonance images. Preferably, the MRI device 100 can perform the MRI on the tissue region of interest at any time, such as before, during, or after a radiotherapy. Preferably, the linear accelerator 200 can perform the radiotherapy on the lesion tissue within the ROI based on fusion images formed by the MRI images and images of other types, thereby further improving the accuracy of the radiotherapy.

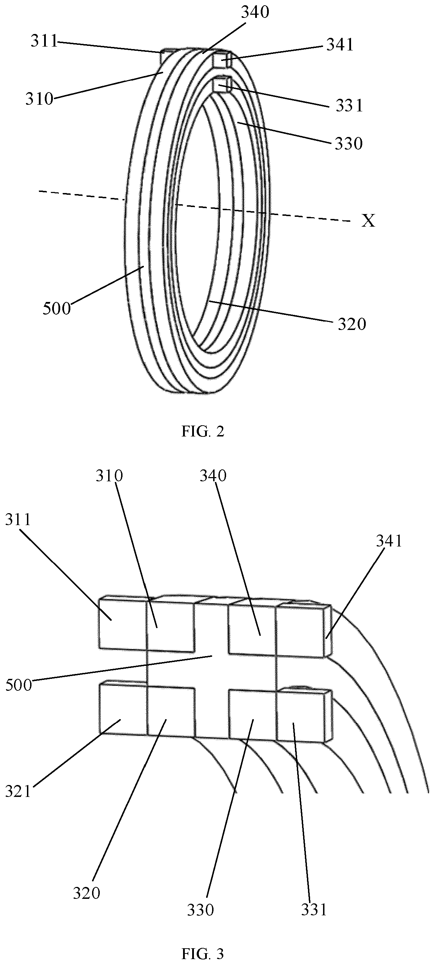

Referring to FIG. 2 and FIG. 3, FIG. 2 is a perspective diagram of a slip ring assembly 300 according to an embodiment of the present application, and FIG. 3 is a schematic sectional view of the slip ring assembly 300 shown in FIG. 2. In the embodiment shown in FIG. 2 and FIG. 3, the slip ring assembly 300 includes: at least one first conductor group and at least one second conductor group. Each first conductor group includes a first conductor 310 and a second conductor 320, and the first conductor 310 and the second conductor 320 are configured as annular structures respectively. Optionally, each first conductor group may include more than two conductors, such as three or four conductors, etc. Each second conductor group includes a third conductor 330 and a fourth conductor 340, and the third conductor 330 and the fourth conductor 340 are configured as annular structures respectively. Optionally, each second conductor group may include more than two conductors, such as three or four conductors, etc. Specifically, in this embodiment, one first conductor group and one second conductor group are provided. Optionally, in other embodiments, more than one first conductor group, for example, two or three first conductor groups, etc., may be arranged; or more than one second conductor group, for example, two or three second conductor groups, etc., may be arranged.

In the embodiment shown in FIG. 2 and FIG. 3, the first conductor group and the second conductor group are arranged at different positions along the axial direction (X). The first conductor 310 and the second conductor 320 are provided at the same position along the axial direction (X), and the third conductor 330 and the fourth conductor 340 are provided at the same position along the axial direction (X). A radial dimension of the first conductor 310 is greater than a radial dimension of the second conductor 320, and a radial dimension of the fourth conductor 340 is greater than a radial dimension of the third conductor 330. Optionally, the radial dimension of the first conductor 310 is less than the radial dimension of the second conductor 320, and the radial dimension of the fourth conductor 340 is less than the radial dimension of the third conductor 330. Preferably, the radial dimension of the first conductor 310 is equal to the radial dimension of the fourth conductor 340, and the radial dimension of the second conductor 320 is equal to the radial dimension of the third conductor 330. It should be noted that the "radial dimension" herein should be broadly understood. For example, the "radial dimension" may be an outer diameter, an inner diameter, or an arithmetic mean of the outer diameter and the inner diameter of an annular body of the annular structure, etc. Optionally, the first conductor group and the second conductor group are arranged coaxially. Optionally, the first conductor 310 and the second conductor 320 are arranged coaxially. Optionally, the third conductor 330 and the fourth conductor 340 are arranged coaxially. Preferably, the first conductor 310, the second conductor 320, the third conductor 330, and the fourth conductor 340 are arranged coaxially. For example, in the embodiment shown in FIG. 2 and FIG. 3, the first conductor 310, the second conductor 320, the third conductor 330, and the fourth conductor 340 are arranged to have a common axis (X).

In the embodiment shown in FIG. 2 and FIG. 3, the first conductor 310, the second conductor 320, the third conductor 330 and the fourth conductor 340 are spaced from each other. For example, an insulator 500 is arranged between the first conductor 310, the second conductor 320, the third conductor 330, and the fourth conductor 340. Optionally, the first conductor 310, the second conductor 320, the third conductor 330, and the fourth conductor 340 may also be spaced apart from each other in other suitable manners. For example, gaps each are formed between the first conductor 310, the second conductor 320, the third conductor 330, and the fourth conductor 340, or electrical isolation materials are filled in the gaps between the first conductor 310, the second conductor 320, the third conductor 330, and the fourth conductor 340, etc., which is not limited in this application.

In the embodiment shown in FIG. 2 and FIG. 3, a projection of the first conductor 310 and a projection of the fourth conductor 340 along the axial direction (X) of the slip ring assembly 300 at least partially overlap, and a projection of the second conductor 320 and a projection of the third conductor 330 along the axial direction (X) of the slip ring assembly 300 at least partially overlap. Preferably, the projection of the first conductor 310 and the projection of the fourth conductor 340 along the axial direction (X) of the slip ring assembly 300 completely overlap, or the projection of the second conductor 320 and the projection of the third conductor 330 along the axial direction (X) of the slip ring assembly 300 completely overlap. More preferably, the projection of the first conductor 310 and the projection of the fourth conductor 340 along the axial direction (X) of the slip ring assembly 300 completely overlap, and the projection of the second conductor 320 and the projection of the third conductor 330 along the axial direction (X) of the slip ring assembly 300 completely overlap.

In the embodiment shown in FIG. 2 and FIG. 3, a first brush 311, a second brush 321, a third brush 331, and a fourth brush 341 are arranged. The first brush 311, the second brush 321, the third brush 331, and the fourth brush 341 are adapted to form electrical contacts with the first conductor 310, the second conductor 320, the third conductor 330, and the fourth conductor 340 respectively. Further, the first brush 311, the second brush 321, the third brush 331, and the fourth brush 341 each are adapted to be in an electrical contact with the electrically connecting terminal 230, so that the slip ring assembly 300 is electrically connected to the treating device 200. Optionally, the first brush 311, the second brush 321, the third brush 331, and the fourth brush 341 may be carbon brushes, metal brushes, or other structures adapted to form electrical contacts, which are not limited in this application.

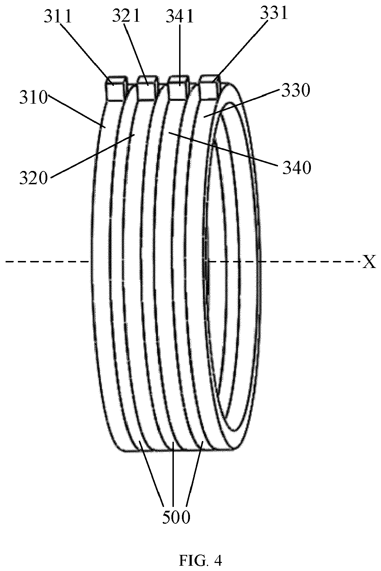

Referring to FIG. 4, FIG.4 is a perspective diagram of the slip ring assembly according to an embodiment of the present application. The embodiment shown in FIG. 4 is approximately similar to the embodiment shown in FIG. 2 and FIG. 3, except the arrangements of the conductor groups and of all conductors. In the embodiment shown in FIG. 4, the first conductor 310 and the second conductor 320 are arranged at different positions along the axial direction(X), and the third conductor 330 and the fourth conductor 340 are arranged at different positions along the axial direction (X). The first conductor 310, the second conductor 320, the fourth conductor 340, and the third conductor 330 are arranged in sequence along the axial direction (X). Optionally, the radial dimension of the first conductor 310 and the radial dimension of the second conductor 320 are the same. Optionally, the radial dimension of the fourth conductor 340 and the radial dimension of the third conductor 330 are the same. Preferably, the first conductor 310, the second conductor 320, the fourth conductor 340, and the third conductor 330 have the same radial dimensions. Optionally, projections of at least two of the first conductor 310, the second conductor 320, the third conductor 330, and the fourth conductor 340 in the axial direction (X) at least partially overlap. Preferably, projections of at least two of the first conductor 310, the second conductor 320, the third conductor 330, and the fourth conductor 340 in the axial direction (X) completely overlap. More preferably, the projections of the first conductor 310, second conductor 320, third conductor 330, and fourth conductor 340 in the axial direction (X) completely overlap.

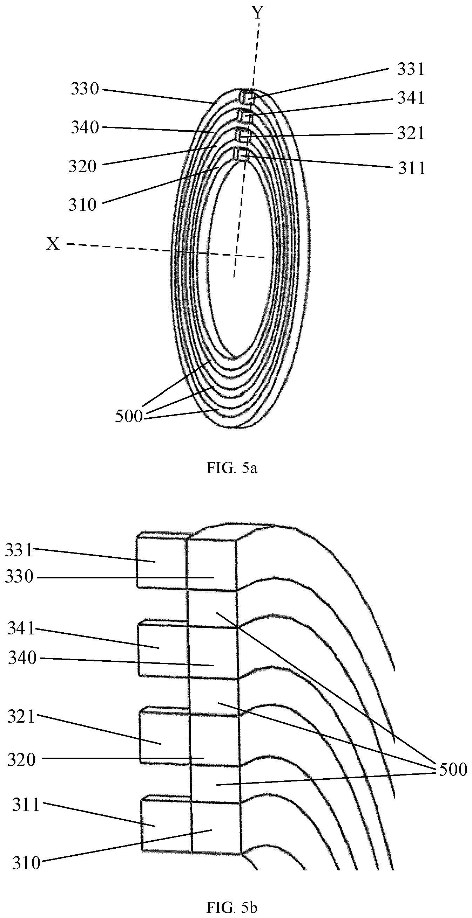

Referring to FIG. 5a and FIG. 5b, FIG. 5a is a perspective diagram of the slip ring assembly according to an embodiment of the present application, and FIG. 5b is a schematic sectional view of the slip ring assembly shown in FIG. 5a. The embodiment shown in FIG. 5a and FIG. 5b is approximately similar to the embodiment shown in FIG. 2 and FIG. 3, except the arrangements of the conductor groups and of all conductors. In the embodiment shown in FIG. 5a and FIG. 5b, the first conductor group and the second conductor group are arranged at the same position along the axial direction (X), the first conductor 310 and the second conductor 320 are arranged at the same position along the axial direction (X), and the third conductor 330 and the fourth conductor 340 are arranged at the same position along the axial direction (X). The radial dimension of the first conductor 310, the radial dimension of the second conductor 320, the radial dimension of the fourth conductor 340, and the radial dimension of the third conductor 330 increase in sequence. Optionally, the radial dimension of the first conductor 310, the radial dimension of the second conductor 320, the radial dimension of the fourth conductor 340, and the radial dimension of the third conductor 330 decrease in sequence. Optionally, the projections of at least two of the first conductor 310, the second conductor 320, the fourth conductor 340, and the third conductor 330 in the radial direction (Y) at least partially overlap. Preferably, the projections of at least two of the first conductor 310, the second conductor 320, the fourth conductor 340, and the third conductor 330 in the radial direction (Y) completely overlap. Preferably, the projections of the first conductor 310, the second conductor 320, the fourth conductor 340, and the third conductor 330 in the radial direction (Y) completely overlap.

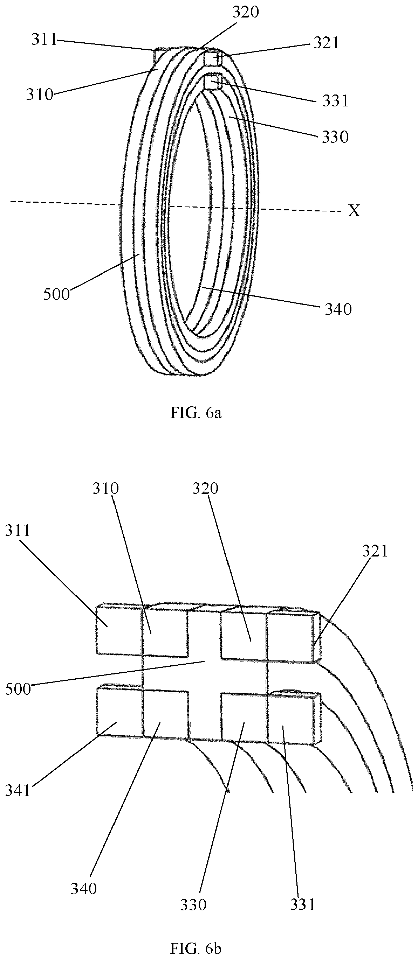

Referring to FIG. 6a and FIG. 6b, FIG. 6a is a perspective diagram of the slip ring assembly according to an embodiment of the present application, and FIG. 6b is a schematic sectional view of the slip ring assembly shown in FIG. 6a. The embodiment shown in FIG. 6a and FIG. 6b are approximately similar to the embodiment shown in FIG. 2 and FIG. 3, except the arrangements of the conductor groups and of all conductors. In the embodiment shown in FIG. 6a and FIG. 6b, the first conductor 310 and the second conductor 320 are arranged at different positions in a first direction along the axis (X), and the fourth conductor 340 and the third conductor (330) are arranged at different positions in the first direction along the axis (X). Please be noted that the first direction herein refers to any one direction along the axis (X). Preferably, the smaller one of the radial dimension of the first conductor 310 and the radial dimension of the second conductor 320 is greater than the larger one of the radial dimension of the third conductor 330 and the radial dimension of the fourth conductor 340. More preferably, the radial dimension of the first conductor 310 is equal to the radial dimension of the second conductor 320, and the radial dimension of the third conductor 330 is equal to the radial dimension of the fourth conductor 340. Optionally, the projections of the first conductor 310 and second conductor 320 in the axial direction (X) at least partially overlap, and the projections of the fourth conductor 340 and third conductor 330 in the axial direction (X) at least partially overlap. Preferably, the projections of the first conductor 310 and second conductor 320 in the axial direction (X) completely overlap, and the projections of the fourth conductor 340 and third conductor 330 in the axial direction (X) completely overlap.

In the embodiments shown in FIG. 2 to FIG. 4, FIG. 5a, FIG. 5b, FIG. 6a, and FIG. 6b, when the slip ring assembly 300 is powered on, a first current having a first phase and a first amplitude flows through the first conductor 310, a second current having a second phase and a second amplitude flows through the second conductor 320, a third current having a third phase and a third amplitude flows through the third conductor 330, and a fourth current having a fourth phase and a fourth amplitude flows through the fourth conductor 340. A difference between the first phase and the third phase is less than or equal to a first threshold, or a difference between the first amplitude and the third amplitude is less than or equal to a second threshold, and a difference between the second phase and the fourth phase is less than or equal to a third threshold, or a difference between the second amplitude and the fourth amplitude is less than or equal to a fourth threshold, so that, after being superposed in space, a first magnetic field generated by the first conductor group and a second magnetic field generated by the second conductor group may at least partially offset each other. Optionally, the first threshold and/or the third threshold may be 20°, 40°, 59°, 60°, 61°, or other suitable values. Preferably, the first threshold and/or the third threshold are 60°. Optionally, the second threshold is twice the smaller one of the first amplitude and the third amplitude, and the fourth threshold is twice the smaller one of the second amplitude and the fourth amplitude. Since after being superposed in space, the first magnetic field generated by the first conductor group and the second magnetic field generated by the second conductor group at least partially offset each other, the overall intensity of the magnetic field (an interference magnetic field) generated in the slip ring assembly 300 is reduced, thereby reducing the interference in the central magnetic field of the MRI device 100 (shown in FIG. 1), and ensuring the accuracy of the MRI.

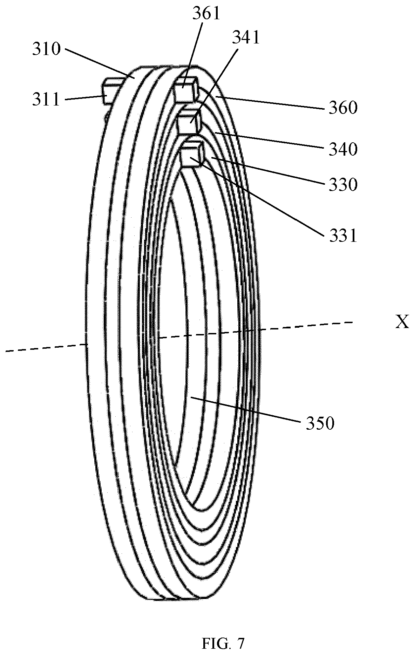

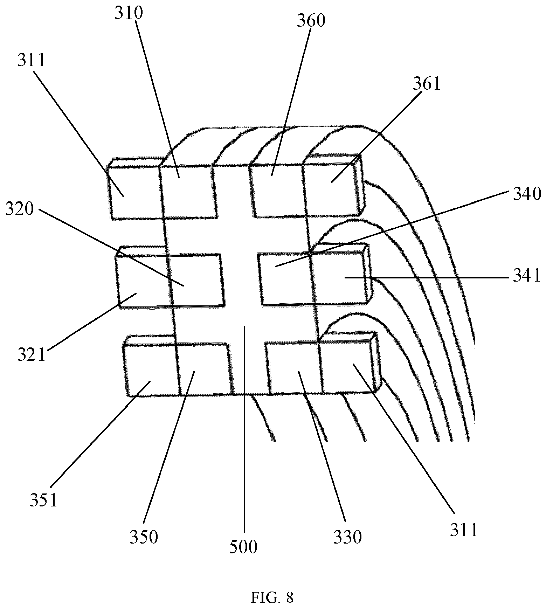

Referring to FIG. 7 and FIG. 8, FIG. 7 is a perspective diagram of a slip ring assembly according to an embodiment of the present application, and FIG. 8 is a schematic sectional view of the slip ring assembly shown in FIG. 7. The first conductor 310, the second conductor 320, the third conductor 330, and the fourth conductor 340 of the embodiment shown in FIG. 7 and FIG. 8 are similar to the embodiment shown in FIG. 2 and FIG. 3. In the embodiment shown in FIG. 7 and FIG. 8, each first conductor group further includes a fifth conductor 350, and the fifth conductor 350 is configured to be in an annular shape. Each second conductor group further includes a sixth conductor 360, and the sixth conductor 360 is configured to be in an annular shape.

In the embodiment shown in FIG. 7 and FIG. 8, the first conductor group and the second conductor group are arranged at the different positions along the axial direction (X). Optionally, at least two of the first conductor 310, the second conductor 320, and the fifth conductor 350 are arranged at the same position along the axis (X), and at least two of the third conductor 330, the fourth conductor 340, and the sixth conductor 360 are arranged at the same position along the axial direction (X). Preferably, the first conductor 310, the second conductor 320, and the fifth conductor 350 are arranged at the same position along the axial direction (X), and the third conductor 330, the fourth conductor 340, and the sixth conductor 360 are arranged at the same position along the axial direction (X). The radial dimension of the first conductor 310, the radial dimension of the second conductor 320, and the radial dimension of the fifth conductor 350 decrease in sequence, and the radial dimension of the sixth conductor 360, the radial dimension of the fourth conductor 340, and the radial dimension of the third conductor 330 decrease in sequence. Preferably, the radial dimension of the first conductor 310 is equal to the radial dimension of the sixth conductor 360, the radial dimension of the second conductor 320 is equal to the radial dimension of the fourth conductor 340, and the radial dimension of the fifth conductor (350) is equal to the radial dimension of third conductor (330). Optionally, the first conductor group and the second conductor group are arranged coaxially. Optionally, the first conductor 310, the second conductor 320, and the fifth conductor 350 are coaxially arranged. Optionally, the third conductor 330, the fourth conductor 340, and the sixth conductor 360 are arranged coaxially. Preferably, the first conductor 310, the second conductor 320, the third conductor 330, the fourth conductor 340, the fifth conductor 350, and the sixth conductor 360 are coaxially arranged. For example, in the embodiment shown in FIG. 7 and FIG. 8, the first conductor 310, the second conductor 320, the third conductor 330, the fourth conductor 340, the fifth conductor 350, and the sixth conductor 360 are arranged to have the common axis (X).

In the embodiment shown in FIG. 7 and FIG. 8, the first conductor 310, the second conductor 320, the third conductor 330, the fourth conductor 340, the fifth conductor 350, and the sixth conductor 360 are arranged to be apart from each other. For example, the insulator 500 is arranged between the first conductor 310, the second conductor 320, the third conductor 330, the fourth conductor 340, the fifth conductor 350, and the sixth conductor 360. Optionally, the first conductor 310, the second conductor 320, the third conductor 330, the fourth conductor 340, the fifth conductor 350, and the sixth conductor 360 may also be arranged to be apart from each other in other suitable manners. For example, gaps are formed between the first conductor 310, the second conductor 320, the third conductor 330, the fourth conductor 340, the fifth conductor 350, and the sixth conductor 360, or electrical isolation materials are filled in the gaps between the first conductor 310, the second conductor 320, the third conductor 330, the fourth conductor 340, the fifth conductor 350, and the sixth conductor 360, etc., which is not limited in this application.

In the embodiment shown in FIG. 7 and FIG. 8, the projections of the first conductor 310 and sixth conductor 360 along the axial direction (X) of the slip ring assembly 300 at least partially overlap, the projections of the second conductor 320 and fourth conductor 340 along the axial direction (X) of the slip ring assembly 300 at least partially overlap, and the projections of the fifth conductor 350 and third conductor 330 along the axial direction (X) of the slip ring assembly 300 at least partially overlap. Preferably, the projections of the first conductor 310 and sixth conductor 360 along the axial direction (X) of the slip ring assembly 300 completely overlap, or the projections of the second conductor 320 and fourth conductor 340 along the axial direction (X) of the slip ring assembly 300 completely overlap, or the projections of the fifth conductor 350 and third conductor 330 along the axial direction (X) of the slip ring assembly 300 completely overlap. More preferably, the projections of the first conductor 310 and sixth conductor 360 along the axial direction (X) of the slip ring assembly 300 completely overlap, and the projections of the second conductor 320 and fourth conductor 340 along the axial direction (X) of the slip ring assembly 300 completely overlap, and the projections of the fifth conductor 350 and third conductor 330 along the axial direction (X) of the slip ring assembly 300 completely overlap.

The first brush 311, the second brush 321, the third brush 331, and the fourth brush 341 of the embodiment shown in FIG. 7 and FIG. 8 are similar to the embodiment shown in FIG. 2 and FIG. 3. The embodiment shown in FIG. 7 and FIG. 8 further include a fifth brush 351 and a sixth brush 361. The fifth brush 351 and the sixth brush 361 are adapted to form electrical contacts with a fifth conductor 350 and a sixth conductor 360 respectively. Further, the fifth brush 351 and the sixth brush 361 are adapted to electrically contact the electrically connecting terminal 230 so that the slip ring assembly 300 is electrically connected to the treating device 200. Optionally, the first brush 311, the second brush 321, the third brush 331, the fourth brush 341, the fifth brush 351, and the sixth brush 361 may be carbon brushes, metal brushes, or other structures suitable for forming electrical contacts, which are not limited in this application.

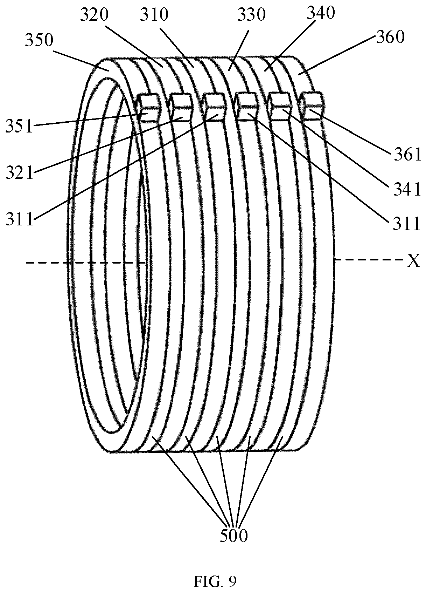

Referring to FIG. 9, FIG. 9 is a perspective diagram of the slip ring assembly according to an embodiment of the present application. The embodiment shown in FIG. 9 is approximately similar to the embodiment shown in FIG. 7 and FIG. 8, except the arrangements of the conductor groups and of all conductors. In the embodiment shown in FIG. 9, optionally, at least two of the first conductor 310, the second conductor 320, and the fifth conductor 350 are arranged at the different positions along the axial direction (X), and at least two of the third conductor 330, the fourth conductor 340, and the sixth conductor 360 are arranged at the different positions along the axial direction (X). Preferably, the first conductor 310, the second conductor 320, and the fifth conductor 350 are arranged at different positions along the axial direction (X), and the third conductor 330, the fourth conductor 340, and the sixth conductor 360 are arranged at different positions along the axial direction (X). The fifth conductor 350, the second conductor 320, the first conductor 310, the third conductor 330, the fourth conductor 340, and the sixth conductor 360 are arranged in sequence along the axial direction (X). Optionally, the radial dimension of the first conductor 310, the radial dimension of the second conductor 320, and the radial dimension of the fifth conductor 350 are the same. Optionally, the radial dimension of the sixth conductor 360, the radial dimension of the fourth conductor 340, and the radial dimension of third conductor 330 are the same. Preferably, the radial dimension of the first conductor 310, the radial dimension of the second conductor 320, the radial dimension of the fifth conductor 350, the radial dimension of the sixth conductor 360, the radial dimension of the fourth conductor 340, and the radial dimension of the third conductor 330 are the same. More preferably, the projections of the first conductor 310, second conductor 320, third conductor 330, fourth conductor 340, fifth conductor 350, and sixth conductor 360 along the axial direction (X) of the slip ring assembly 300 completely overlap.

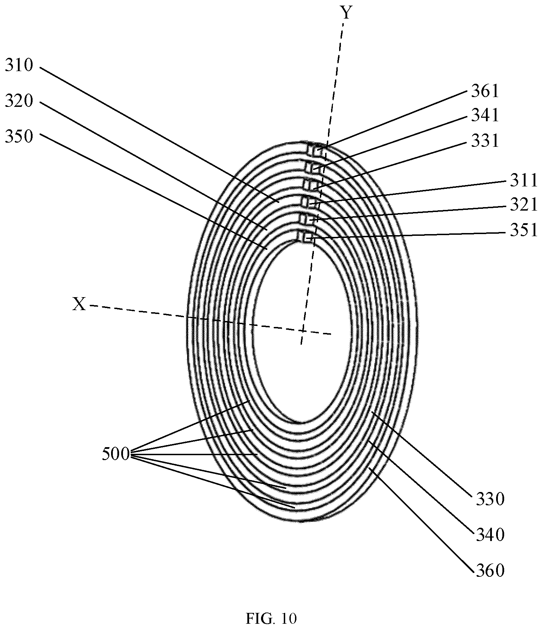

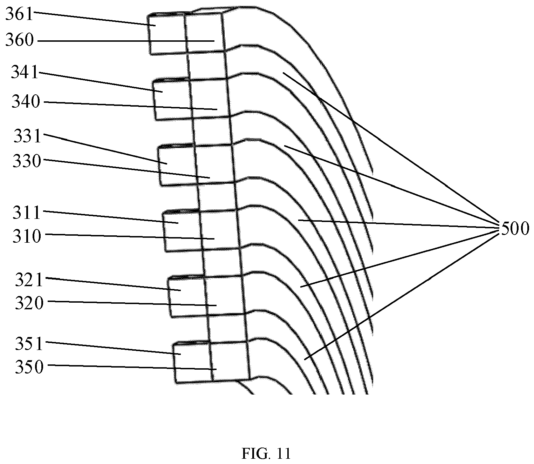

Referring to FIG. 10 and FIG. 11, FIG. 10 is a perspective diagram of the slip ring assembly according to an embodiment of the present application, and FIG. 11 is a schematic sectional view of the slip ring assembly shown in FIG. 10. The embodiment shown in FIG. 10 and FIG. 11 are approximately similar to the embodiment shown in FIG. 7 and FIG. 8, except the arrangements of the conductor groups and of all conductors. In the embodiment shown in FIG. 10 and FIG. 11, the first conductor group and the second conductor group are arranged at the same position along the axial direction (X). Optionally, at least two of the first conductor 310, the second conductor 320 and the fifth conductor 350 are arranged at the same position along the axial direction (X), and at least two of the third conductor 330, the fourth conductor 340 and the sixth conductor 360 are arranged at the same position along the axial direction (X). Preferably, the first conductor 310, the second conductor 320, and the fifth conductor 350 are arranged at the same position along the axial direction (X), and the third conductor 330, the fourth conductor 340 and the sixth conductor 360 are arranged at the same position along the axial direction (X). The radial dimension of the fifth conductor 350, the radial dimension of the second conductor 320, the radial dimension of the first conductor 310, the radial dimension of the third conductor 330, the radial dimension of the fourth conductor 340, and the radial dimension of the sixth conductor 360 increase in sequence. Optionally, the radial dimension of the fifth conductor 350, the radial dimension of the second conductor 320, the radial dimension of the first conductor 310, the radial dimension of the third conductor 330, the radial dimension of the fourth conductor 340, and the radial dimension of the sixth conductor 360 decrease in sequence. Optionally, the projections of at least two of the first conductor 310, second conductor 320, third conductor 330, fourth conductor 340, fifth conductor 350, and sixth conductor 370 in the radial direction (Y) at least partially overlap. Preferably, the projections of at least two of the first conductor 310, second conductor 320, third conductor 330, fourth conductor 340, fifth conductor 350, and sixth conductor 370 in the radial direction (Y) completely overlap. Preferably, the projections of the first conductor 310, second conductor 320, third conductor 330, fourth conductor 340, fifth conductor 350, and sixth conductor 370in the radial direction (Y) completely overlap.

In another embodiment according to the present application, the first conductor group and the second conductor group are arranged at the same position along the axial direction (X). Optionally, at least two of the first conductor 310, the second conductor 320, and the fifth conductor 350 are arranged at different positions along the axial direction (X), and at least two of the third conductor 330, the fourth conductor 340, and the sixth conductor 360 are arranged at different positions along the axial direction (X). Preferably, the first conductor 310, the second conductor 320, and the fifth conductor 350 are arranged at different positions in the second direction along the axis (X), and the third conductor 330, the fourth conductor 340, and the sixth conductor 360 are arranged at different positions in the second direction along the axis (X). Please be noted that the first direction in this text refers to any direction along the axial direction (X). Optionally, the smallest one of the radial dimension of the first conductor 310, the radial dimension of the second conductor 320, and the radial dimension of the fifth conductor 350 is greater than the largest one of the radial dimension of the third conductor 330, the radial dimension of the fourth conductor 340, and the radial dimension of the sixth conductor (360). Preferably, the first conductor 310, the second conductor 320, and the fifth conductor 350 have the same radial dimension. More preferably, the projections of the first conductor 310, second conductor 320, and fifth conductor 350 in the axial direction (X) completely overlap. Preferably, the third conductor 330, the fourth conductor 340, and the sixth conductor (360) have the same radial dimension. More preferably, the projections of the third conductor 330, fourth conductor 340, and sixth conductor (360) in the axial direction (X) completely overlap.

In the embodiments shown in FIG. 7 to FIG. 11, the first current, the second current, the third current, the fourth current, the first phase, the second phase, the third phase, the fourth phase, the first amplitude, the second amplitude, the third amplitude, the fourth amplitude, and the first threshold, the second threshold, the third threshold, and the fourth threshold are approximately identical with the embodiment shown in FIG. 2 and FIG. 3. In the embodiments shown in FIG. 7 to FIG. 11, when the slip ring assembly 300 is powered on, a fifth current having a fifth phase and a fifth amplitude flows through the fifth conductor 350, and a sixth current having a sixth phase and a sixth amplitude flows through the sixth conductor 360. A difference between the fifth phase and the sixth phase is less than or equal to a fifth threshold, or a difference between the fifth amplitude and the sixth amplitude is less than or equal to a sixth threshold, so that, after being superposed in space, the first magnetic field generated by the first conductor group and the second magnetic field generated by the second conductor group at least partially offset each other. Optionally, the fifth threshold may be 20°, 40°, 59°, 60°, 61°, or any other suitable value. Preferably, the fifth threshold is 60°. Optionally, the sixth threshold is twice the smaller one of the fifth amplitude and the sixth amplitude. Since after being superposed in space, the first magnetic field generated by the first conductor group and the second magnetic field generated by the second conductor group at least partially offset each other, the overall intensity of the magnetic field (the interference magnetic field) generated in the slip ring assembly 300 is reduced, thereby further reducing the interference in the central magnetic field of the MRI device 100 (shown in FIG. 1), and ensuring the accuracy of the MRI.

The technical features of the embodiments above may be combined arbitrarily. To make the description concise, not all possible combinations of the technical features in the above embodiments are described. However, as long as there are no contradictions in the combinations of these technical features, all of the combinations should be considered to be within the scope of the specification.

The embodiments above only represent several implementation modes of the present application, and the description thereof is relatively specific and detailed, but it should not be construed as limiting the scope of the patent. It should be noted that for those skilled in the art, various modifications and improvements may be made without departing from the concept of the present application, and all these modifications and improvements belong to the protection scope of the present application. Therefore, the scope of protection of the patent application should be subject to the appended claims.

## Claims

1. A slip ring assembly (300), **characterized by** comprising:
at least one first conductor group, each first conductor group comprising a first conductor (310) and a second conductor (320), and the first conductor (310) and the second conductor (320) being configured as annular structures, respectively; and
at least one second conductor group, each second conductor group comprising a third conductor (330) and a fourth conductor (340), the third conductor (330) and the fourth conductor (340) being configured as annular structures, respectively;
wherein the first conductor (310), the second conductor (320), the third conductor (330) and the fourth conductor (340) are arranged to be apart from each other;
a first current having a first phase and a first amplitude is configured to flow through the first conductor (310), a second current having a second phase and a second amplitude is configured to flow through the second conductor (320), a third current having a third phase and a third amplitude is configured to flow through the third conductor (330), and a fourth current having a fourth phase and a fourth amplitude is configured to flow through the fourth conductor (320);
a difference between the first phase and the third phase is less than or equal to a first threshold, and/or a difference between the first amplitude and the third amplitude is less than or equal to a second threshold;
a difference between the second phase and the fourth phase is less than or equal to a third threshold, and/or a difference between the second amplitude and the fourth amplitude is less than or equal to a fourth threshold; and
a first magnetic field generated by the first conductor group and a second magnetic field generated by the second conductor group at least partially offset each other.

2. The slip ring assembly (300) according to claim 1, wherein the first threshold and/or the third threshold are60°.

3. The slip ring assembly (300) according to claim 1, wherein the second threshold is twice a smaller one of the first amplitude and the third amplitude, and/or the fourth threshold is twice a smaller one of the second amplitude and the fourth amplitude.

4. The slip ring assembly (300) according to claim 1, wherein the first conductor group and the second conductor group are coaxially arranged.

5. The slip ring assembly (300) according to claim 1, wherein the first conductor (310) and the second conductor (320) are coaxially arranged.

6. The slip ring assembly (300) according to claim 1, wherein the third conductor (330) and the fourth conductor (340) are coaxially arranged.

7. The slip ring assembly (300) according to any one of claims 1 to 6, wherein the first conductor group and the second conductor group are arranged at different positions along an axial direction (X).

8. The slip ring assembly (300) according to claim 7, wherein the first conductor (310) and the second conductor (320) are arranged at the same position along the axial direction (X), and the third conductor (330) and the fourth conductor (340) are arranged at the same position along the axial direction (X).

9. The slip ring assembly (300) according to claim 8, wherein a radial dimension of the first conductor (310) is greater than a radial dimension of the second conductor (320), and a radial dimension of the fourth conductor (340) is greater than a radial dimension of the third conductor (330).

10. The slip ring assembly (300) according to claim 9, wherein the radial dimension of the first conductor (310) is equal to the radial dimension of the fourth conductor (340), and the radial dimension of the second conductor (320) is equal to the radial dimension of the third conductor (330).

11. The slip ring assembly (300) according to claim 7, wherein the first conductor (310) and the second conductor (320) are arranged at different positions along the axial direction (X), and the third conductor (330) and the fourth conductor (340) are arranged at different positions along the axial direction (X).

12. The slip ring assembly (300) according to claim 11, wherein the first conductor (310), the second conductor (320), the fourth conductor (340), and the third conductor (330) are arranged in sequence along the axial direction (X).

13. The slip ring assembly (300) according to claim 12, wherein a radial dimension of the first conductor (310), a radial dimension of the second conductor (320), a radial dimension of the fourth conductor (340) and a radial dimension of the third conductor (330) are the same.

14. The slip ring assembly (300) according to any one of the preceding claims 1 to 6, wherein the first conductor group and the second conductor group are arranged at the same position along an axial direction (X).

15. The slip ring assembly (300) according to claim 14, wherein the first conductor (310) and the second conductor (320) are arranged at the same position along the axial direction (X), and the third conductor (330) and the fourth conductor (340) are arranged at the same position along the axial direction (X).

16. The slip ring assembly (300) according to claim 15, wherein a radial dimension of the first conductor (310), a radial dimension of the second conductor (320), a radial dimension of the fourth conductor (340), and a radial dimension of the third conductor (330) increase in sequence.

17. The slip ring assembly (300) according to claim 14, wherein the first conductor (310) and the second conductor (320) are arranged at different positions in a first direction along an axis (X), and the fourth conductor (340) and the third conductor (330) are arranged at different positions in the first direction along the axis (X).

18. The slip ring assembly (300) according to claim 17, wherein a smaller one of a radial dimension of the first conductor (310) and a radial dimension of the second conductor (320) is greater than a larger one of a radial dimension of the third conductor (330) and a radial dimension of the fourth conductor (340).

19. The slip ring assembly (300) according to claim 18, wherein the radial dimension of the first conductor (310) is equal to the radial dimension of the second conductor (320), and the radial dimension of the third conductor (330) is equal to the radial dimension of the fourth conductor (340).

20. The slip ring assembly (300) according to any one of claims 1 to 6, wherein the first conductor group further comprises a fifth conductor (350), and the fifth conductor (350) is configured to be in an annular shape; the second conductor group further comprises a sixth conductor (360), and the sixth conductor (360) is configured to be in an annular shape;
the first conductor (310), the second conductor (320), the third conductor (330), the fourth conductor (340), the fifth conductor (350), and the sixth conductor (360) are configured to be apart from each other;
a fifth current having a fifth phase and a fifth amplitude is configured to pass through the fifth conductor (330), and a sixth current having a sixth phase and a sixth amplitude is configured to pass through the sixth conductor (360);
a difference between the fifth phase and the sixth phase is less than or equal to a fifth threshold, or a difference between the fifth amplitude and the sixth amplitude is less than or equal to a sixth threshold.

21. The slip ring assembly (300) according to claim 20, wherein the fifth threshold is 60°.

22. The slip ring assembly (300) according to claim 20, wherein the sixth threshold is twice a smaller one of the fifth amplitude and the sixth amplitude.

23. The slip ring assembly (300) according to claim 20, wherein the first conductor (310), the second conductor (320) and the fifth conductor (350) are coaxially arranged.

24. The slip ring assembly (300) according to claim 20, wherein the third conductor (330), the fourth conductor (340), and the sixth conductor (360) are coaxially arranged.

25. The slip ring assembly (300) according to any one of claims 20 to 24, wherein at least two of the first conductor (310), the second conductor (320) and the fifth conductor (350) are arranged at the same position along an axial direction (X), and at least two of the third conductor (330), the fourth conductor (340) and the sixth conductor (360) are arranged at the same position along the axial direction (X).

26. The slip ring assembly (300) according to claim 25, wherein the first conductor (310), the second conductor (320) and the fifth conductor (350) are arranged at the same position along the axial direction (X), and the third conductor (330), the fourth conductor (340) and the sixth conductor (360) are arranged at the same position along the axial direction (X).

27. The slip ring assembly (300) according to claim 26, wherein a radial dimension of the first conductor (310), a radial dimension of the second conductor (320) and a radial dimension of the fifth conductor (350) decrease in sequence, and a radial dimension of the sixth conductor (360), a radial dimension of the fourth conductor (340), and a radial dimension of the third conductor (330) decrease in sequence.

28. The slip ring assembly (300) according to claim 27, wherein the radial dimension of the first conductor (310) is equal to the radial dimension of the sixth conductor (360), the radial dimension of the second conductor (320) is equal to the radial dimension of the fourth conductor (340), and the radial dimension of the fifth conductor (350) is equal to the radial dimension of the third conductor (330).

29. The slip ring assembly (300) according to any one of claims 20 to 24, wherein at least two of the first conductor (310), the second conductor (320) and the fifth conductor (350) are arranged at different positions along an axial direction (X), and at least two of the third conductor (330), the fourth conductor (340) and the sixth conductor (360) are arranged different positions along the axial direction (X).

30. The slip ring assembly (300) according to claim 29, wherein the first conductor (310), the second conductor (320) and the fifth conductor (350) are arranged at different positions along the axial direction (X), and the third conductor (330), the fourth conductor (340) and the sixth conductor (360) are arranged at different positions along the axial direction (X).

31. The slip ring assembly (300) according to claim 30, wherein the fifth conductor (350), the second conductor (320), the first conductor (310), the third conductor (330), the fourth conductor (340) and the sixth conductor (360) are arranged in sequence along the axial direction (X).

32. The slip ring assembly (300) according to any one of claims 20 to 24, wherein at least two of the first conductor (310), the second conductor (320) and the fifth conductor (350) are arranged at the same position along an axial direction (X), and at least two of the third conductor (330), the fourth conductor (340) and the sixth conductor (360) are at the same position along the axial direction (X).

33. The slip ring assembly (300) according to claim 32, wherein the first conductor (310), the second conductor (320) and the fifth conductor (350) are arranged at the same position along the axial direction (X), the third conductor (330), the fourth conductor (340) and the sixth conductor (360) are arranged at the same position along the axial direction (X).

34. The slip ring assembly (300) according to claim 33, wherein a radial dimension of the fifth conductor (350), a radial dimension of the second conductor (320), a radial dimension of the first conductor (310), a radial dimension of the third conductor (330), a radial dimension of the fourth conductor (340), and a radial dimension of the sixth conductor (360) increase in sequence.

35. The slip ring assembly (300) according to any one of claims 20 to 24, wherein at least two of the first conductor (310), the second conductor (320) and the fifth conductor (350) are arranged at different positions along an axial direction (X), and at least two of the third conductor (330), the fourth conductor (340) and the sixth conductor (360) are arranged at different positions along the axial direction (X).

36. The slip ring assembly (300) according to claim 35, wherein the first conductor (310), the second conductor (320) and the fifth conductor (350) are arranged at different positions in a second direction along the axial direction (X); and the third conductor (330), the fourth conductor (340) and the sixth conductor (360) are arranged at different positions in the second direction along the axial direction (X).

37. The slip ring assembly (300) according to claim 36, wherein the smallest one of a radial dimension of the first conductor (310), a radial dimension of the second conductor (320) and a radial dimension of the fifth conductor (350) is greater than the largest one of a radial dimension of the third conductor (330), a radial dimension of the fourth conductor (340), and a radial dimension of the sixth conductor (360).

38. The slip ring assembly (300) according to any one of the preceding claims, further comprising a first brush (311), a second brush (321), a third brush (331) and a fourth brush (341), the first brush (311) is adapted to form an electrical contact with the first conductor (310), the second brush (321) is adapted to form an electrical contact with the second conductor (320), the third brush (331) is adapted to form an electrical contact with the third conductor (330), and the fourth brush (341) is adapted to form an electrical contact with the fourth conductor (340).

39. The slip ring assembly (300) according to any one of claims 20 to 38, further comprising a fifth brush (351) and a sixth brush (361), wherein the fifth brush (351) is adapted to form an electrical contact with the fifth conductor (350), and the sixth brush (361) is adapted to form an electrical contact with the sixth conductor (360).

40. A medical device (10), comprising a treating device (200), **characterized in that** the treating device (200) comprises the slip ring assembly (300) according to any one of claims 1 to 39, and the slip ring assembly (300) is adapted to act as a power supply medium or a communication medium of the treating device (200).

41. The medical device (10) according to claim 40, further comprising a magnetic resonance imaging device (100).
